# EUROPÄISCHE PATENTSCHRIFT

(11) **EP 2 943 144 B1**
(45) Veröffentlichungstag und Bekanntmachung des Hinweises auf die Patenterteilung: **21.02.2018**
(21) Anmeldenummer: 13830180.9
(22) Anmeldetag: 27.12.2013
(51) Int. Cl.: A61C 13/00, A61C 13/34

(54) **VERFAHREN ZUR HERSTELLUNG EINES GEBISSMODELLS MITTELS EINER FRÄSMASCHINE**
METHOD FOR PRODUCING A DENTAL WORKING MODEL BY MEANS OF A MILLING MACHINE
PROCÉDÉ DE FABRICATION D'UN MODÈLE DE TRAVAIL DENTAIRE AU MOYEN D'UNE FRAISEUSE

(30) Priorität: 28.12.2012 DE 102012025218
(43) Veröffentlichungstag der Anmeldung: 18.11.2015
(73) Patentinhaber: Feldmann, Harald, 55288 Armsheim (DE)
(72) Erfinder: Feldmann, Harald, 55288 Armsheim (DE)
(74) Vertreter: Kodron, Felix
(86) Internationale Anmeldenummer: PCT/DE2013/100442
(87) Internationale Veröffentlichungsnummer: WO 2014/101917

(56) Entgegenhaltungen:
- WO-A1-2011/103879
- US-A1- 2006 127 858
- US-A1- 2009 130 634
- US-A1- 2011 196 524

## Beschreibung

Die Erfindung betrifft ein Verfahren zur Herstellung eines Gebißmodells mittels einer Fräßmaschine gemäß den Merkmalen des Anspruchs 1 sowie hierfür verwendbares Werkstück. Es ist im Stand der Technik bekannt, zur Herstellung eines Gebißmodells bei der Fertigung von Zahnersatz digitale Daten zur bildgebenden Erfassung der Gebißsituation im Kiefer eines Patienten zu verwenden. Diese Daten werden mittels moderner Scannervorrichtungen und -verfahren im Mundraum eines Patienten erfasst und durch spezielle Softwareanwendungen zu Modellen der Kiefersituation eines Patienten umgewandelt. Auf diese Weise ist es möglich, EDV-gestützte Modelle für die Herstellung von Zahnersatz bzw. für die Herstellung der Kiefermodelle zu schaffen.

Es ist hierbei bekannt, aus verschiedenen Werkstoffen mittels beispielsweise CNC-Fräsmaschinen Gebißmodelle zu fertigen. Hierzu ist auch die Verwendung von Kunststoffen bekannt, aus denen die CNC-Fräsmaschinen die entsprechenden am Rechner entworfenen Gebißmodelle herausfräsen. Es ist hierbei üblich, diese Gebissmodelle bzw. Zahnkränze auf Sockelplatten aufzustecken, um so die Zahnkränze bearbeiten zu können und diese auch in Artikulatoren einsetzen zu können. Es ist ebenfalls bekanntes Vorgehen, die Zahnkränze zur Bearbeitung in Segmente zu zersägen, um exaktere Arbeiten an diesen Einzelsegmenten vornehmen zu können.

Die in Segmente zerteilten Zahnkranzmodelle müssen aber auch später zur Prüfung der Modelle im Artikulator zusammengesetzt werden können, weshalb in Sockelplatten Bohrungen zur Aufnahme von Haltestiften vorgesehen sind. Diese Haltestifte dienen der Verbindung der Zahnkranzsegmente mit der diese tragenden Sockelplatte, wofür die Gebißmodelle in Ihrer Unterseite eine Vielzahl von korrespondierenden Bohrungen aufweisen müssen, in die die Haltestifte eingreifen. So kann auf der Sockelplatte das Gebißmodell nachgestellt werden.

In der Praxis fallen nun verschiedene Arbeitsvorgänge an um diese Verbindung zwischen einer Sockelplatte und dem Gebißmodell zu schaffen. Zum einen wird das Gebißmodell in der zuvor beschriebenen Form über einen Scannvorgang und einen Fräsvorgang erstellt, wobei auch die Position der Implantate im Kiefer mit zu erfassen ist.

Die Sockelplatte ist in der Regel ein separates zugekauftes Produkt, das dann in Verbindung mit dem Gebissmodell zu bringen ist. Hierfür ist beispielsweise bei der Fertigung eines Gebißmodells aus einem Kunststoffkörper ein Einbringen entsprechender Bohrungen in der Unterseite des Gebißmodells erforderlich, um fluchtende Bohrungen im Gebißmodell wie auch in der Sockelplatte zu haben, um dann die Steckverbindung zwischen Sockelplatte und Gebißmodell herstellen zu können.

In der Praxis handelt es sich hierbei also um einen weiteren vergleichsweise aufwendigen Verfahrensvorgang, wobei die Anordnung der Bohrungen in der Unterseite des Gebißmodels exakt mit den Bohrungen in der Sockelplatte übereinstimmen muss, damit ein Aufstecken des Gebißmodells auf die Sockelplatte ohne ein Verkanten auf den Haltestiften möglich ist. Erfahrungsgemäß kommt es hierbei immer wieder zu Problemen, wenn es hier zu Abweichungen in den Bohrungen kommt, da so die Positionen der einzelnen Gebisssegmente auf der Sockelplatte nicht exakt ausgerichtet werden kann.

Aus der Veröffentlichung US 2009/130634 A1 ist ein Cluster Fräsrohling umfassend einen Rahmen bekannt, um mit einer Halterung eines vorhandenen CAD / CAM-Systems zusammenarbeiten, wobei eine Vielzahl von Teilrohlingen im Rahmen angeordnet sind und eine adressierbare Matrix bilden.

Aus der Veröffentlichung US 2006/127858 A1 ist ein System und ein Verfahren zur Herstellung einer Basis für Zahnmodelle bekannt, wobei das Erfassen der Koordinaten der Zahnmodelle im Kiefermodell unter Verwendung einer optischen Positionsvorrichtung erfolgt. Hierbei werden Bohrungen zur Verbindung einer Sockelplatte mir dem Zahnkranzmodell vorgenommen.

Vor diesem Hintergrund ist es die Aufgabe der vorliegenden Erfindung, eine Vereinfachung bei der Herstellung eines Gebißmodell sowie der Anordnung dieses Gebißmodells auf einer Sockelplatte zu bewirken. Gleichzeitig soll eine exakte Ausrichtung des Gebißmodells auf der Sockelplatte ermöglicht und eine Reduzierung der Herstellungskosten erreicht werden.

Erreicht wird dies nach der Erfindung durch das Verfahren mit den kennzeichnenden Merkmalen des Anspruchs 1.

Anspruch 5 betrifft ein Werkstück zur Verwendung in diesem Verfahren.

Die weiteren Ansprüche haben vorteilhafte Gestaltungen der Erfindung zum Gegenstand.

Die Kernidee des erfinderischen Vorgehens liegt darin, die Herstellung eines Gebissmodells mit der Herstellung eines entsprechenden Sockelplatte verfahrenstechnisch zu verknüpfen. Wird ein Gebißmodell beispielsweise in einer CNC-Fräsmaschine hergestellt, so ist der erfinderische Gedanke, gleichzeitig mit dem Gebißmodell auch eine Sockelplatte in dieser CNC-Fräsmaschine mit herzustellen, sodass Gebißmodell und Sockelplatte als ein Herstellungsprozeß zu sehen sind, der ein perfekt aufeinander abgestimmtes Produkt erzeugt.

Hierbei ist der erfinderische Vorgang darin begründet, dass nicht wie im üblichen Verfahren ein einzelnes Werkstück, beispielsweise eine Kunststoff-Scheibe bzw. -Ronde, in die Fräsmaschine eingespannt und der entsprechenden formgebenden Fräsbehandlung unterzogen wird, sondern dass zwei dieser Werkstücke in die Fräsmaschine eingespannt werden. Diese zwei scheibenförmigen Körper werden hierbei durch den Einspannvorgang in die Fräsmaschine so miteinander verbunden, dass eine Bearbeitung als ein Werkstück möglich ist.

Beim Bearbeiten dieser Kunststoff-Ronden wird somit aus der ersten Hälfte dieses Kombinationswerkstücks der Zahnkranz herausgefräst mit entsprechenden Aufnahmen für die Implantate. Aus dem zweiten Element des Werkstücks wird ein Sockel herausgefräst, wobei die Bohrungen zur Aufnahme der Haltestifte direkt durch diese Sockelscheibe in die Unterseite des Zahnkranzes hineingebohrt werden. Auf diese Weise ist eine absolute Passgenauigkeit sichergestellt, da die durchgängigen Bohrungen durch die Sockelplatte sowie die Sacklochbohrungen in der Unterseite des Zahnkranzes in einem Bohrvorgang hergestellt werden.

Es ist hierbei eine vorteilhafte Gestaltung des Verfahrens sowie des im Verfahren verwendeten Werkstücks, dass die miteinander bearbeiteten Werskstückteile durch eine entsprechende Formgebung verdrehsicher miteinander verbunden sind. Es ist hierbei beispielsweise eine Nut- und Federverbindung beispielsweise im Randbereich der Kunststoffronden eine mögliche Ausgestaltung der Werkstückteile, die dazu beiträgt, dass ein Verdrehen der der Werkstoffteile gegeneinander während des eingespannten Fräsvorgangs ausgeschlossen ist.

Eine weitere vorteilhafte Gestaltung der Erfindung ist darin zu sehen, dass in den gefrästen Zahnkranz an der durch den Scannvorgang festgelegten Position des Implantatkörpers eine standardisierte Aufnahmebohrung eingebracht wird, in die eine spezielle Adapterhülse eingesetzt werden kann. Hierbei ist der erfinderische Ansatz, dass spezielle Adapterhülsen für unterschiedliche Implantatfabrikate vorgesehen sind, die dadurch gekennzeichnet sind, dass alle Adapterhülsen in Ihrer Aussenkontur identisch sind und somit in die standardisierte gefräste Aufnahme für die Adapterhülse im Zahnkranzmodell eingesetzt werden können.

Das heißt, die Aufnahme im Zahnkranzmodell kann immer standardisiert gleich eingefräst werden, da die Wahl der Hülse, die dann in diese Aufnahme eingesteckt werden kann eine Aufnahme des entsprechenden Zahnimplantats eines bestimmten Herstellers ermöglicht, da die Innenkontur der Aufnahme in der Hülse an diese unterschiedlichen Implantatkörper angepaßt ist. So kann in das Zahnmodell mittels der eingesetzten Adapterhülse genau das Zahhnimplantat für die Modelierung des Implantats bzw. Zahnerssatzes eingesetzt werden, dass auch in der tatsächlichen Kiefersituation des Patienten vorliegt.

Hierin ist eine wesentlicher Vorteil gegenüber Verfahren zu sehen, bei denen die Gebißmodelle nachträglich eingesetzte Implantate aufweisen, die dann in Ihrer Position nicht 100 % der Situation im Gebiß des Patienten entsprechen.

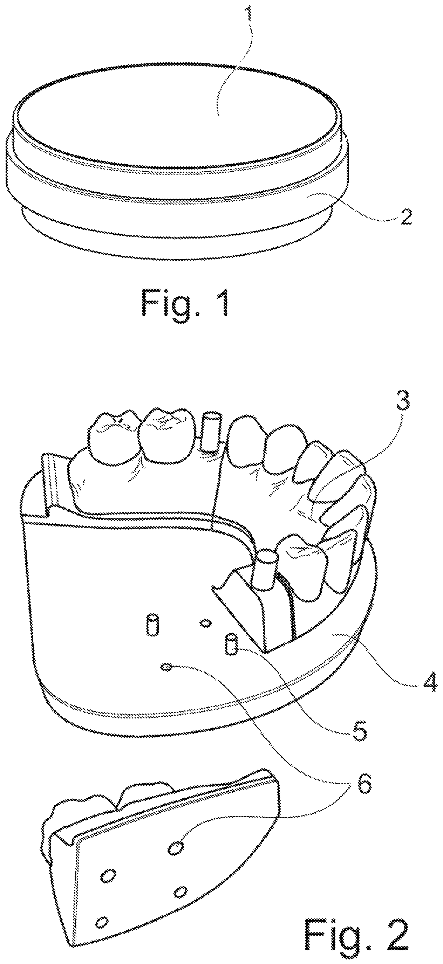

Nachfolgend ist das erfinderische Verfahren anhand von Abbildungen näher erläutert. Es zeigt Figur 1 die Werkstückkombination bestehend aus zwei aufeinander aufliegenden Ronden 1 und 2, die in der dargestellten Bauform Scheiben gleichen Durchmessers sind. Hierbei ist die Dicke der verwendeten Ronden 1 und 2 im Anwendungsfall unterschiedlich, da die Sockelplatte, auf die der Zahnkranz aufgesteckt werden soll, eine geringere Stärke aufweisen muss, als dies für den Zahnkranz der Fall ist. In Figur 1 handelt es sich hierbei um die noch unbearbeiteten Körper, bevor hier eine Verbindung erfolgt.

Figur 2 wiederum zeigt die Verbindung aus einem ausgefrästen Gebißmodell 3 und der entsprechenden Sockelplatte 4 mit Haltestiften 5 zur Verbindung des Gebißmodells 3 auf dieser Sockelplatte 4. Die exakte Abstimmung der Anordnung der Bohrung in dieser Sockelplatte 4 zum Gebißmodell 3 ist hierbei entscheidend.

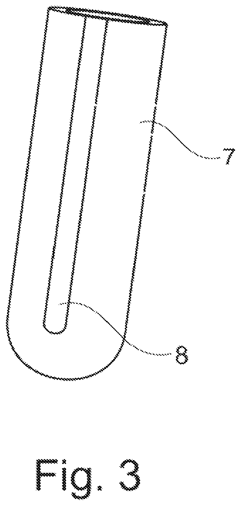

In Figur 3 ist eine Adapterhülse 7 dargestellt, die in eine standardisierte Aufnahmebohrung des Gebißmodells 3 einsetzbar ist. Hierbei betrifft die Standardisierung die Abstimmung der in das Gebißmodell 3 eingebrachten Bohrung mit der Außenkontur der Adapterhülse 7, so daß die in ihrer Außenkontur immer gleich gestalteten Adapterhülsen immer in diese Aufnahmen eingesetzt werden können. Lediglich die Innenkontur der Adapterhülsen ist abgestimmt auf die verschiedenen Ausbildungen der Implantatkörper unterschiedlicher Hersteller, wodurch diese nach Einsetzen der entsprechenden Adapterhülse in das Gebißmodell einsetzbar sind. Eine Nut 8 an der Außenseite der Adapterhülse sichert diese im Zusammenwirken mit einer Feder in der Aufnahme gegen ein Verdrehen im Gebißmodell.

Zusammenfassend ist festzustellen, dass die besonderen Vorteile nicht nur in der exakten Bearbeitung von Gebißmodell und Sockelplatte in einem Arbeitsvorgang zu sehen sind, sondern auch im Zusammenführen der Herstellung der Sockelplatte mit der Herstellung des Gebißmodells. Auf diese Weise ist es möglich, in einer Werkstatt bzw. in einem Verfahrensgang beide Bauteile aus dem selben Material zu produzieren und das Auseinanderfallen bei der Herstellung von Sockelplatte einerseits und individuellem Gebißmodell andererseits zu überwinden. Auch was Kostenentwicklungen im Bereich der Implantatmedizin anbetrifft birgt dies einige Vorteile.

## Patentansprüche

1. Verfahren zur Herstellung eines Gebißmodells (3) mit einer Fräsmaschine, wobei hierfür digitale Daten eines Scannvorgang der Gebißsituation beim Patienten zur EDV-gestützten Modellierung eines Gebißmodells (3) herangezogen werden, **dadurch gekennzeichnet, dass**
- für den Fräsvorgang ein Werkstück aus zumindest zwei Werkstückteilen (1, 2) lösbar zusammengesetzt in eine Fräsmaschine eingespannt wird,
- wobei eines der Werkstückteile (2) zur Herstellung des Zahnkranzes des Gebißmodells (3) und zumindest ein zweites Werkstückteil (1) zur gleichzeitigen Herstellung einer Sockelplatte (4) verwendet wird
- und die Bohrungen (6) zur Verbindung von Sockelplatte (4) und Zahnkranz in einem Arbeitsvorgang durch die Sockelplatte (4) in die Unterseite des Gebißmodells (3) erfolgen.

2. Verfahren zur Herstellung eines Gebißmodells (3) mit einer Fräsmaschine nach Anspruch 1,
**dadurch gekennzeichnet, dass**
die zumindest zwei Werkstückteile (1, 2) zur Einspannung in die Fräsmaschine mittels einer formschlüssigen Verbindung zwischen den aneinander anliegenden Werkstückteilen verdrehsicher miteinander verbunden sind.

3. Verfahren zur Herstellung eines Gebißmodells (3) mit einer Fräsmaschine nach Anspruch 1 oder 2,
**dadurch gekennzeichnet, dass**
beim Fräsvorgang in den gefrästen Zahnkranz an der beim Scannvorgang erfassten Position eines Implantatkörpers eine standardisierte Aufnahmebohrung eingebracht wird, in die eine spezielle in ihrer Außenkontur standardisierte Adapterhülse für das verwendete Implantat eingesetzt wird.

4. Verfahren zur Herstellung eines Gebißmodells (3) mit einer Fräsmaschine nach Anspruch 3,
**dadurch gekennzeichnet, dass**
- die speziellen Adapterhülsen Aufnahmen angepasst für unterschiedliche Implantatfabrikate aufweisen,
- wobei alle Adapterhülsen in Ihrer Aussenkontur identisch sind und somit in die standardisierte gefräste Aufnahme für die Adapterhülse im Zahnkranzmodell eingesetzt werden können.

5. Werkstück aus zumindest zwei Werkstückteilen (1, 2) zur Herstellung eines Gebißmodells (3) mit einer Fräsmaschine nach dem Verfahren nach Anspruch 1,
**dadurch gekennzeichnet, dass**
die zumindest zwei Werkstückteile (1, 2) mittels zumindest einer Vertiefung und zumindest einem in diese Vertiefung eingreifenden Vorsprung formschlüssig aber lösbar miteinander verbunden sind, wodurch die aneinander anliegenden Werkstückteile verdrehsicher in die Fräsmaschine einspannbar sind.

## Claims

1. Method for producing a dentition model (3) by means of a milling machine, for which purpose digital data from a scanning operation of the dental situation presented by the patient are used for EDP-assisted modelling of a dentition model (3), **characterized in that**
- a workpiece composed of at least two workpiece parts (1, 2) is clamped in detachably assembled fashion in a milling machine for the milling operation,
- one of the workpiece parts (2) is used to produce the toothed rim of the dentition model (3) and at least one second workpiece part (1) is used to simultaneously produce a base plate (4),
- and the bores (6) for connecting base plate (4) and toothed rim are produced in one work operation through the base plate (4) into the underside of the dentition model (3).

2. Method for producing a dentition model (3) by means of a milling machine according to Claim 1, **characterized in that** the at least two workpiece parts (1, 2) for clamping in the milling machine are connected to each other, in a manner secure against rotation, by means of a form-fit connection between the mutually adjoining workpiece parts.

3. Method for producing a dentition model (3) by means of a milling machine according to Claim 1 or 2, **characterized in that**, in the milling operation, a standardized receiving bore is introduced into the milled toothed rim, at the implant body position detected in the scanning operation, and a special adapter sleeve with a standardized outer contour for the implant used is inserted into the receiving bore.

4. Method for producing a dentition model (3) by means of a milling machine according to Claim 3, **characterized in that**
- the special adapter sleeves have seats adapted to different implant products,
- wherein all of the adapter sleeves are identical in terms of their outer contour and can thus be inserted into the standardized, milled seat for the adapter sleeve in the model of the toothed rim.

5. Workpiece composed of at least two workpiece parts (1, 2) for producing a dentition model (3) by means of a milling machine in the method according to Claim 1, **characterized in that** the at least two workpiece parts (1, 2) are connected to each other with a form fit, but in a detachable manner, by means of at least one depression and at least one projection engaging in this depression, as a result of which the mutually adjoining workpiece parts can be clamped in the milling machine in a manner secure against rotation.

## Revendications

1. Procédé de fabrication d'un modèle de dentition (3) avec une fraiseuse, des données numériques d'une opération de balayage des conditions dentaires du patient étant utilisées à cet effet pour une modélisation informatique d'un modèle de dentition (3),
**caractérisé en ce que**
- pour l'opération de fraisage, une pièce assemblée de manière détachable à partir d'au moins deux parties de pièce (1, 2) est serrée dans une fraiseuse,
- l'une des parties de pièce (2) est utilisée pour fabriquer la couronne dentaire du modèle de dentition (3) et au moins une deuxième partie de pièce (1) est utilisée pour la fabrication simultanée d'une plaque de socle (4),
- et les perçages (6) pour la connexion de la plaque de socle (4) et de la couronne dentaire sont effectués dans une opération de travail à travers la plaque de socle (4) dans le côté inférieur du modèle de dentition (3).

2. Procédé de fabrication d'un modèle de dentition (3) avec une fraiseuse selon la revendication 1,
**caractérisé en ce que**
les au moins deux parties de pièce (1, 2) pour le serrage dans la fraiseuse sont connectées l'une à l'autre de manière solidaire en rotation au moyen d'une liaison par engagement par correspondance de formes entre les parties de pièce s'appliquant l'une contre l'autre.

3. Procédé de fabrication d'un modèle de dentition (3) avec une fraiseuse selon la revendication 1 ou 2,
**caractérisé en ce que**
lors de l'opération de fraisage, un perçage de réception standardisé est réalisé dans la couronne dentaire fraisée au niveau de la position d'un corps d'implant détectée lors de l'opération de balayage, dans lequel perçage de réception est insérée une douille d'adaptateur spéciale de contour extérieur standard pour l'implant utilisé.

4. Procédé de fabrication d'un modèle de dentition (3) avec une fraiseuse selon la revendication 3,
**caractérisé en ce que**
- les douilles d'adaptateur spéciales présentent des logements adaptés à des fabrications d'implants différentes,
- toutes les douilles d'adaptateur ayant un contour extérieur identique et pouvant être de ce fait insérées dans le logement fraisé standardisé pour la douille d'adaptateur dans le modèle de couronne dentaire.

5. Pièce constituée d'au moins deux parties de pièce (1, 2) pour la fabrication d'un modèle de dentition (3) avec une fraiseuse selon le procédé de la revendication 1,
**caractérisée en ce que**
les au moins deux parties de pièce (1, 2) sont connectées par engagement par correspondance de formes mais de manière détachable l'une à l'autre au moyen d'au moins un renfoncement et d'au moins une saillie s'engageant dans ce renfoncement, de sorte que les parties de pièce s'appliquant l'une contre l'autre puissent être serrées dans la fraiseuse de manière solidaire en rotation.
